# EUROPEAN PATENT APPLICATION

(11) **EP 1 480 153 A2**
(43) Date of publication of application: **24.11.2004**
(21) Application number: 04011657.6
(22) Date of filing: 17.05.2004
(51) Int. Cl.: G06F 19/00

(54) **Medical tool mangement and support system**

(30) Priority: 20.05.2003 JP 2003141660
(71) Applicant: Olympus Corporation, Shibuya-ku, Tokyo (JP)
(72) Inventor: Oishi, Hiroko, Hachioji-shi Tokyo 193-0832 (JP); Hirose, Koichi, Hino-shi Tokyo 191-0011 (JP)
(74) Representative: von Hellfeld, Axel, Dr. Dipl.-Phys.

(57) **Abstract**

A medical system for managing a medical tool includes: an identification information obtaining unit (3) for obtaining identification information about a medical tool stored in information resources provided for the medical tool; a medical practice information obtaining unit (1) for obtaining medical practice information about the medical practice performed using the medical tool; and a storage control unit (4) for storing medical tool and practice related information which refers to the association between the identification information obtained from the identification information obtaining unit (1) and the medical practice information obtained from the medical practice information obtaining unit (1).

## Description

### Cross Reference to Related Application

This application is based on and claims the benefit of priority from the prior Japanese Patent Application No. 2003-141660 filed in Japan on May. 20, 2003, the entire contents of which are incorporated by this reference.

### Background of the Invention

### Field of the Invention

The present invention relates to a system and a method for managing tools for use in a medical location, and storage medium, etc. used thereof.

### Description of the Related Art

Recently, the problem of nosocomial infection has become more and more serious, and the protection against nosocomial infection and the method of preventing the spread of nosocomial infection have received widespread attention. For example, one of the method for preventing the spread of nosocomial infection is the follow-up management of the tools used in the medical practice for an examination, treatment, etc. (for example, Japanese Patent Application Laid-open No. 2002-28132). In Japanese Patent Application Laid-open No. 2002-28132, an endoscope used in the medical practice is cleaned (for sterilization and disinfection) and managed, thereby preventing the erroneous use of an uncleaned endoscope.

Thus, the management of medical tools has become more important, and the improvement of the reliability of the management has been demanded. Therefore, for example, when an endoscopic examination is made, the name of the user of a scope and a treatment tool, and the model name and number of the tool with which the treatment tool has been used are recorded before or after using the treatment tool in case of the occurrence of a medical malpractice, nosocomial infection, and a demand for insurance. As a result, in the hospital, the model name and the serial number of a scope and a treatment tool used by a doctor or a nurse in the examination and treatment have been handwritten in a report. If there is the possibility of infection through a scope, a patient having the possibility of infection has been traced based on the serial number and the model name of the scope described in the report.

When a disposable treatment tool is used, the seal attached to the treatment tool is added to the report in requesting the treatment tool used in the examination to be checked.

Thus, by describing and adding the serial number, etc. of a scope and a treatment tool to a report, the treatment tool and the scope used in examination can be correctly traced when a medical malpractice, etc. occurs.

Furthermore, when an indwelling treatment tool is used, the indwelling treatment tool is to be taken off in a predetermined period. However, a patient has to go to the facilities in which the indwelling treatment tool is taken out, and the period is up to the memory of the patient.

### Summary of the Invention

A medical system for managing a medical tool includes:
an identification information obtaining unit for obtaining identification information about a medical tool stored in information resources provided for the medical tool;
a medical practice information obtaining unit for obtaining medical practice information about the medical practice performed using the medical tool; and
a storage control unit for storing medical tool and practice related information which refers to the association between the identification information obtained from the identification information obtaining unit and the medical practice information obtained from the medical practice information obtaining unit.

A medical server system for managing a medical tool according to the present invention includes:
a storage unit for storing medical tool and practice related information about the association between the information about the medical tool and the information about the medical practice performed using the medical tool; and
a notification unit for notification of the expiration date of the medical tool in advance according to the medical tool and practice related information stored in the storage unit.

A medical method for managing a medical tool according to the present invention performs:
obtaining identification information about a medical tool stored in information resources provided for the medical tool;
obtaining medical practice information about the medical practice performed using the medical tool; and
storing medical tool and practice related information which refers to the association between the identification information and the medical practice information.

A computer-readable portable storage medium storing a medical program used to direct a computer to perform the process for managing a medical tool according to the present invention includes:
a identification information obtaining process of obtaining identification information about a medical tool stored in information resources provided for the medical tool;
a medical practice information obtaining process of obtaining medical practice information about the medical practice performed using the medical tool; and
a storage control process of storing medical tool and practice related information which refers to the association between the identification information obtained from the identification information obtaining process and the medical practice information obtained from the medical practice information obtaining process.

A computer data signal embodied in a carrier wave for managing a medical tool according to the present invention used to direct a computer to perform:
a identification information obtaining process of obtaining identification information about a medical tool stored in information resources provided for the medical tool;
a medical practice information obtaining process of obtaining medical practice information about the medical practice performed using the medical tool; and
a storage control process of storing medical tool and practice related information which refers to the association between the identification information obtained from the identification information obtaining process and the medical practice information obtained from the medical practice information obtaining process.

A medical program product used to direct a computer to manage a medical tool according to the present invention includes:
a identification information obtaining process of obtaining identification information about a medical tool stored in information resources provided for the medical tool;
a medical practice information obtaining process of obtaining medical practice information about the medical practice performed using the medical tool; and
a storage control process of storing medical tool and practice related information which refers to the association between the identification information obtained from the identification information obtaining process and the medical practice information obtained from the medical practice information obtaining process.

### Brief Description of the Drawings

FIG. 1 shows the outline of the configuration of the endoscope information system according to the first embodiment of the present invention;
FIG. 2 shows the outline of the basic configuration of the body of the PC according to the first embodiment of the present invention;
FIG. 3A shows an ID tag set in the treatment tool according to the first embodiment of the present invention;
FIG. 3B shows the ID tag set in the scope according to the first embodiment of the present invention;
FIG. 3C shows the ID tag set in the indwelling treatment tool according to the first embodiment of the present invention;
FIG. 4 shows the flow of an entry process for entering the intrinsic information about the write protect ID tag in a server according to the first embodiment of the present invention;
FIG. 5 shows the outline of the configuration of the window of the image filing device according to the first embodiment of the present invention;
FIG. 6 shows the flow of the entire process according to the first embodiment of the present invention;
FIG. 7 shows the flow of the process of generating a nurse report according to the first embodiment of the present invention;
FIG. 8 shows the nurse report (during examination/treatment) generation window according to the first embodiment of the present invention;
FIG. 9 shows the nurse report (after examination/treatment) generation window according to the first embodiment of the present invention;
FIG. 10 shows the ET history window according to the first embodiment of the present invention;
FIG. 11 shows the flow of the indwelling treatment of the indwelling treatment tool according to the second embodiment of the present invention;
FIG. 12 shows the flow of setting the indwelling date by a server according to the second embodiment of the present invention;
FIG. 13 shows the window on which the indwelling period and the relative number of weeks is set according to the second embodiment of the present invention; and
FIG. 14 shows the flow of the process of transmitting reminder mail on the user notification date according to the second embodiment of the present invention.

### Description of the Preferred Embodiments

According to the first embodiment of the present invention, the information about the scope and treatment tool used in and together with the information about the examination or the treatment is easily input and entered in real time during examination or during treatment. Conventionally, the information about a scope, etc. has been manually input. According to the present embodiment it is input and entered in a mechanical operation.

According to the second embodiment, the information entered in the first embodiment is effectively used. That is, the takeoff date of the indwelling treatment tool is automatically announced to a doctor, a nurse, a patient, etc.

Described below is each of the embodiments according to the present invention.

### <First Embodiment>

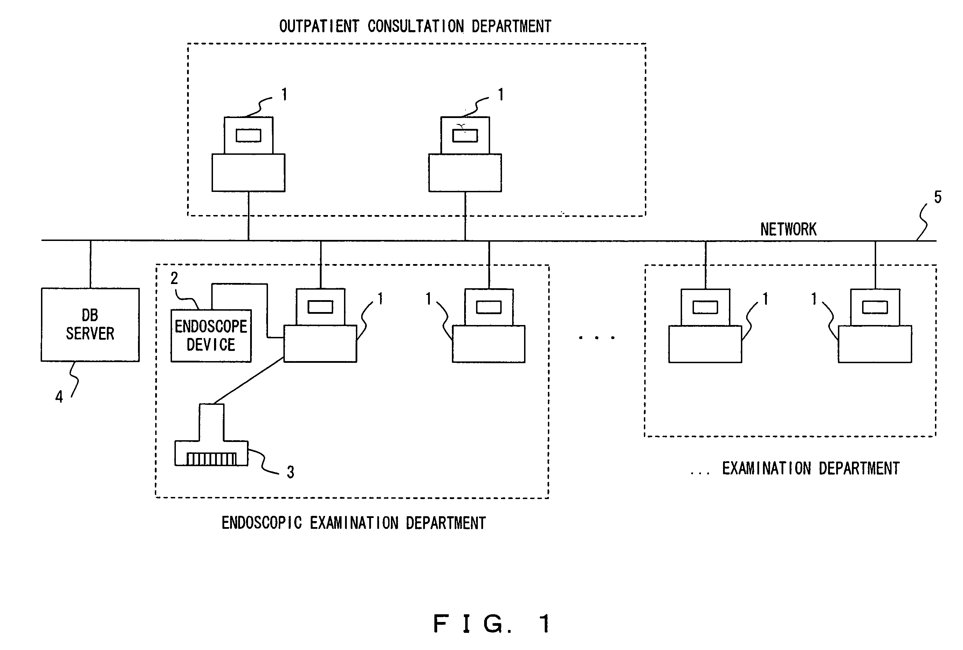

FIG. 1 shows the outline of the configuration of the endoscope information system according to the present embodiment. As shown in FIG. 1, the present system has various departments such as an outpatient consultation department, an endoscopic examination department, etc., and each department has one or more personal computers (hereinafter referred to as a PC) 1. The PC 1 is connected over a network such as a LAN 5 (local area network), etc., and each PC 1 has an input device and an output device.

A database server 4 is connected to the network, and stores patient information, treatment information, etc. input from each PC 1. In the present embodiment, an endoscopic examination department is explained. The PC 1 of the endoscopic examination department is used as an image filing device. An image filing device 1 is connected to an endoscope device 2 and an identification information detection device (ID detector) 3 for detecting identification information. The endoscope device 2 comprises an endoscope (videoscope, and hereinafter referred to as an endoscope or a scope) which is inserted into coelom and captures an examined region, a light source device for supplying illuminating light to the endoscope, an image generation device for processing a capture signal from the endoscope and generating an endoscopic image, a monitor for displaying the endoscopic image generated by the image generation device, a keyboard for inputting data, etc.

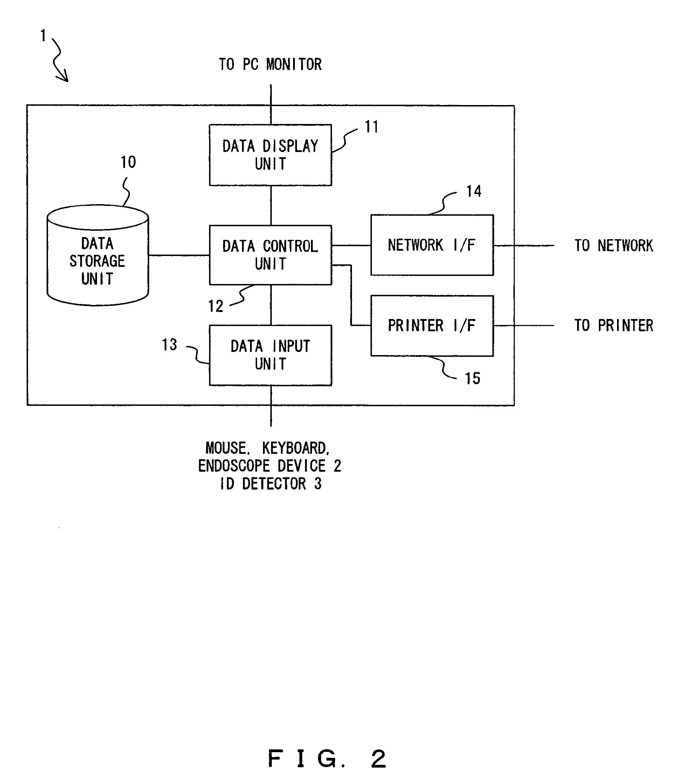

FIG. 2 shows the outline of the basic configuration of the body of each PC 1. The body of the PC 1 comprises, a data control unit (CPU) 12, a data storage unit 10 storing various data such as image data, patient information, treatment information, etc., a data display unit 11 for displaying image data, etc. on a PC monitor, a data input unit 13 inputting an instruction, data, etc. from an input device such as a mouse, a keyboard, the endoscope device 2, the ID detector 3, etc., a network I/F (interface) 14 as an interface for connection to a network such as the LAN 5, etc., and a printer I/F (interface) 15 as an interface for output of data to a printer.

Described below is the ID detector 3 which is one of the input devices. The ID detector 3 detects predetermined identification information (ID). In the present embodiment, an RFID system is used to detect an ID. An RFID system (radio frequency identification) reads and writes information of a transponder (tag, ID tag, or an RFID tag) through electromagnetic induction from a reader/writer without contact.

A transponder (ID tag) refers to a very small and lightweight batteryless communications unit comprising a semiconductor having a transmission-reception circuit, a control circuit, and memory on a chip, and an antenna. Upon receipt of an inquiry radio wave from a reader, the transponder uses this as electric energy, and transmits the information stored in the memory as an electric response wave.

The reader (ID detector) 3 has a built-in control circuit including a microprocessor, controls an antenna and a transmission-reception circuit, demodulates received data, and interfaces with the personal computer. At this time, the ID detector 3 can be connected with the body of the PC 1 via cable or by wireless.

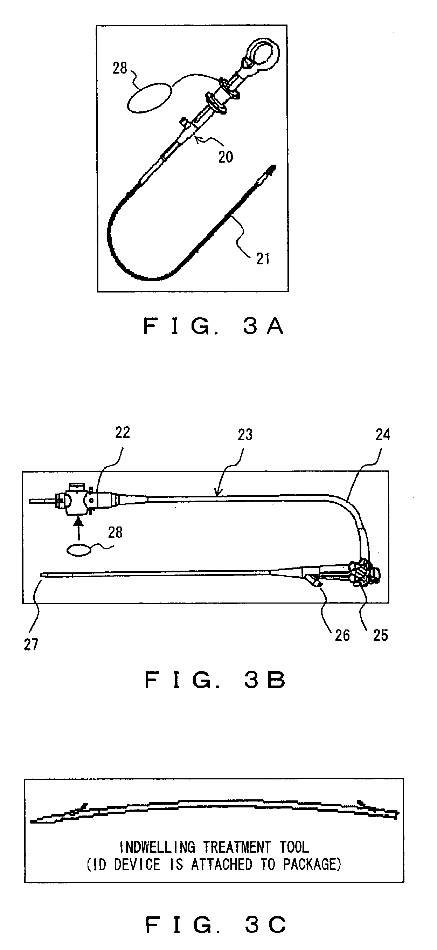

FIGS. 3A, 3B, and 3C show the ID tags set in the treatment tool, the scope, and the indwelling treatment tool respectively. A scope 23 shown in FIG. 3B comprises a coupling unit 22 connected to a video processor, a universal code 24, an operation unit 25 for operating the scope 23, a forceps entrance 26, and a CCD (charge coupled device) 27 for capturing a capture target. A treatment tool 20 shown in FIG. 3A performs treatment while checking an endoscopic image by inserting an insertion unit 21 through the forceps entrance 26.

Each of the treatment tool 20 and the scope 23 is provided with an ID tag 28, and can read the information stored in the ID tag 28 using the ID detector 3.

The indwelling treatment tool shown in FIG. 3C is inserted into a vein and a bile duct to hold their diameters in a predetermined size or expand them. Therefore, the size is the order of the vein and the bile duct, very small, and the ID tag cannot be directly attached. Therefore, in this case, an ID tag is attached to the package of the treatment tool, and the information about the ID tag is read using the ID detector 3.

The information stored in the ID tag according to the present embodiment is explained. First, the ID tag can be either written by a writer to its built-in memory (writable ID tag) or cannot be written (write protect ID tag).

A write protect ID tag stores unique information (for example, information by alphanumeric characters, etc.) by the RFID tag production manufacturer in advance, and the information cannot be changed. When such a write protect ID tag is used, a server stores in advance the unique information, the information about the scope or treatment tool corresponding to the unique information (for example, the production number (serial number), the model name, etc. of the scope or the treatment tool). Therefore, when the unique information stored in the ID tag attached to the scope or the treatment tool is read using the ID detector 3, the server 4 can obtain the information (for example, the serial number, the model name, etc. of the scope or the treatment tool) about the scope or treatment tool corresponding to the unique information, thereby uniquely recognizing the scope or the treatment tool to which the ID is assigned.

The writable ID tag can allow information to be arbitrarily written to its built-in memory. Therefore, the serial number and the model name of the scope or the treatment tool, and the manufacturer unique number of the scope or the treatment tool can be written directly to the memory of the ID tag. In this case, the information about the scope or the treatment tool can be referred to by a client (image filing device 1) without asking the server the information read by the ID detector 3.

Thus, using a writable or write protect ID tag, the serial number, the model name, etc. of the scope or the treatment tool to which the ID is assigned can be discriminated. The above-mentioned information about the scope or the treatment tool stored in the ID tag is hereinafter referred to as intrinsic information.

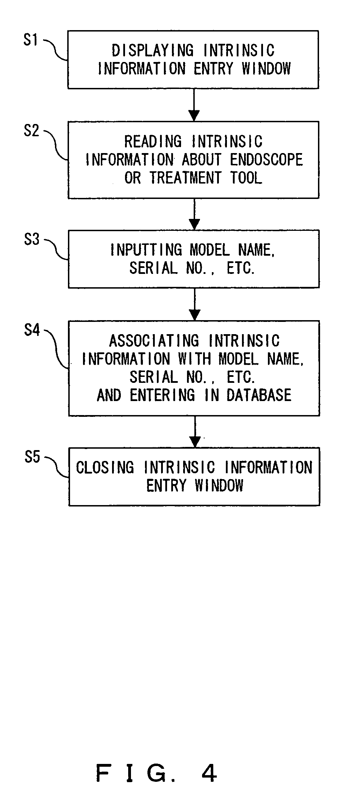

FIG. 4 shows the flow for entering the intrinsic information about a write protect ID tag in a server. First, a write protect ID tag is attached to the scope or the treatment tool. Then, if the intrinsic information entry system using the application program is activated on the PC of another subsystem (for example, a tool management system, etc.), the data control unit of the PC reads the application program, and displays an intrinsic information entry window on the display of the PC (in step S1, and hereinafter a step is referred to simply as S).

The information stored in the attached ID tag is read using a reader (S2). The read unique information about the ID tag is transmitted to the PC through the reader, and displayed in a predetermined item of the intrinsic information entry window. Then, according to the unique information displayed in the predetermined item, the intrinsic information (model name, serial number, etc.) about the scope or the treatment tool to which the ID tag is input (S3).

Then, the read unique information about the ID tag is associated with the intrinsic information about the scope or the treatment tool input corresponding to the unique information, and is entered in the database of the server (S4). The server stores the associated information in a predetermined area of the storage device. After the completion of the entry, the intrinsic information entry window is closed, and the flow terminates (S5). In the process above, the inputting process is performed by the PC, but it can also be performed by the server.

When the intrinsic information about the writable ID tag is entered, the intrinsic information (model name, serial number, manufacturer name, etc.) about the scope or the treatment tool is input using the PC, and the information is written for the ID tag attached to the scope or the treatment tool corresponding to the intrinsic information. The information similar to the written information is entered in the database of the server so that the server can manage it. The server shown in FIG. 4 can be the server 4, or a server of another subsystem.

Described above is the ID tag and the information stored in the ID tag, and described below is an image filing system.

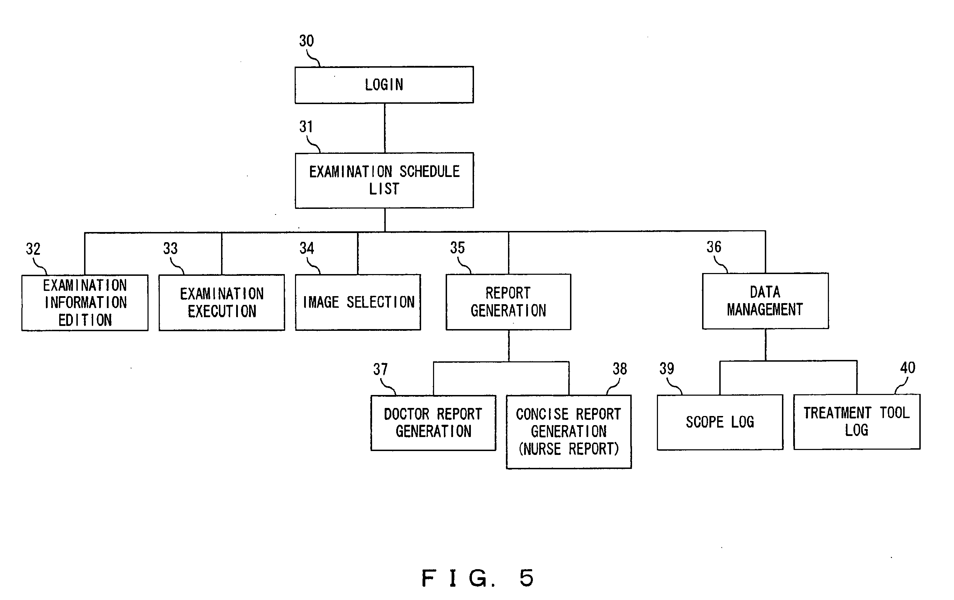

FIG. 5 shows the outline of the configuration of the window of the image filing device 1. First, when the image filing device 1 is activated, the data control unit 12 reads the image filing system program. Then, a login window 30 for authentication of the operator is displayed on the display of the image filing device 1. On the login window 30, the operator inputs his or her user ID and password. The image filing device 1 asks the server whether or not the input user ID and the password are authorized.

The server determines whether or not the user ID and the password match the user ID and password entered in advance. If the determination indicates a matching result, the user is authenticated as an authorized use. When the operator is authenticated as an authorized user, the user can log in to the system, and a schedule list window 31 displaying a list of examination schedules is displayed on the display.

On the schedule list window 31, the examination information list indicating what an examination is made when and on who (patient) using which scope (for example, a scope for an upper region, a scope for a lower region, etc.) is displayed.

On the schedule list window 31, an examination information edition window 32 can be called to newly enter examination information, reserve an examination, and edit already entered examination information.

Also on the schedule list window 31, an examination execution window 33 can be called. The examination execution window 33 is used during examination or during treatment. An image of an affected part captured using the endoscope device 2 connected to the image filing device 1 can be entered in the filing device or the server on the examination execution window 33. The contents of an examination or treatment can also be entered. Furthermore, the information about the ID tag attached to the scope or the treatment tool used at this time is read using the ID detector 3, and the information is temporarily stored in the image filing device 1.

On the schedule list window 31, an image selection window 34 for selection of an image in the generated examination report in the images captured on the examination execution window 33 can be called.

Furthermore, on the schedule list window 31, a report generation window 35 for generation of an examination report can be called. Additionally, the report generation window 35 can make a transition to a doctor report generation window 37 for generation of an examination report by a doctor, or a concise report (nurse report) generation window 38 for generation of an examination report by a nurse. A concise report is generated before treatment, during treatment, and after treatment, and the information about a scope and a treatment tool, and the information about a patient (treatment contents, conditions of a patient, etc.) can be input on the window. The observation of a doctor report is input to a doctor report after treatment.

The schedule list window 31 can make a transition to a data management window 36. On the data management window 36, a scope log window 39 for display of the log of a scope or a treatment tool log window for display of the log of a treatment tool can be selected for transition.

On the scope log window 39, the history information (log) relating to a scope about what scope has been used, when and how the cleaning program is used, etc. can be referred to. On a treatment tool log window 40, the information about an indwelling treatment tool and the information about a reused treatment tool can be referred to.

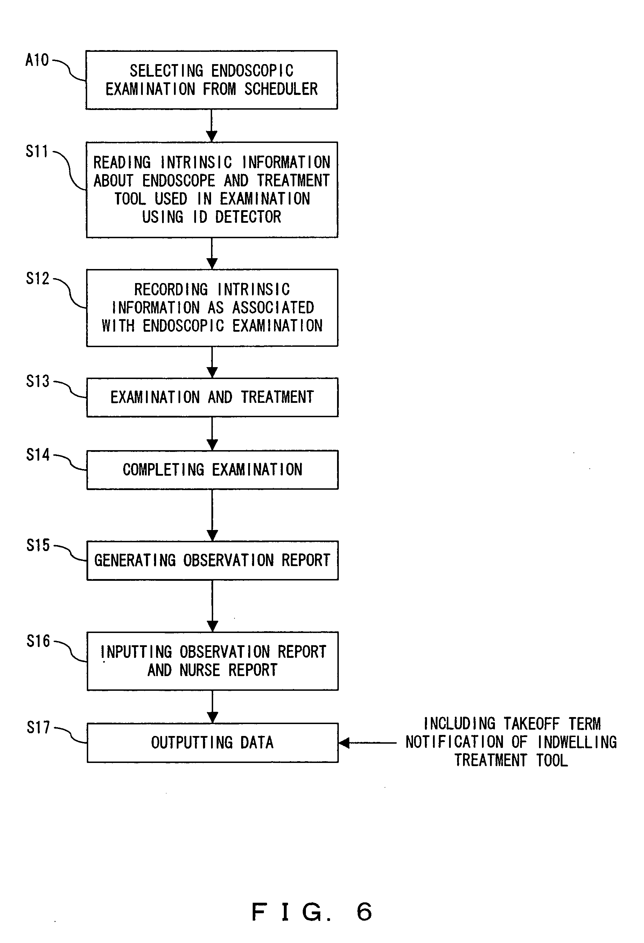

FIG. 6 shows the flow of the entire process of the present system. First, an endoscopic examination is selected on the examination schedule list window 31 (S10), and a transition is made to the examination execution window 33. On the examination execution window 33, the information about the patient relating to the examination and the treatment, that is, the examination information, is input. Furthermore, the intrinsic information about a scope or a treatment tool used in a examination and treatment is read from each ID tag attached to the scope or the treatment tool by the ID detector 3 (S11).

The intrinsic information read by the ID detector 3 is transmitted to the image filing device 1, associated with the examination information (such as patient ID, examination date, examination starting time, etc.), and stored in the data storage unit 10 in the image filing device 1 (S12).

Then, the examination and treatment are actually performed (S13), thereby terminating the examination (S14). When the examination is completed, the examination information input on the examination execution window 33 is entered in the server 4. In this example, the information (information about examination information associated with intrinsic information which is hereinafter referred to as related information) stored in the data storage unit 10 in S12 is transmitted from the image filing device 1 to the server 4. The server 4 receives the information, and the information is stored in the storage device of the server under the control of the CPU of the server.

The timing of entering and updating the information in the server 4 is not limited to the above-mentioned timing. For example, in the system of entering a captured image in the server each time an image is captured by a scope, the image can be entered, and simultaneously the related information can be entered in the server. Thus, the related information can be entered in the server when the server performs the entry or update process with any timing.

Next, an observation report is generated (S15). On the report generation window 35, when an operator is a doctor who performs treatment, "generating a doctor report" is selected on the report generation window 35 to make a transition to the doctor report generation window 37. When an operator is a nurse, "generating a nurse report" is selected on the report generation window 35 to make a transition to the nurse report generation window 38. On each window, the information about the examination and the treatment is input (S16). The information input in S16 is entered in the server 4.

On the nurse report generation window 38, the information before treatment can be input at the stage before S11 to generate a nurse report before examination and treatment, or the information during examination and treatment in S13 can be input to generate a nurse report during examination and treatment.

Afterwards, the data of the information input in S16 is output and effectively utilized (S17). For example, it is transmitted to another system used in the hospital, and used by the system, output on a printer and used, or can be used for notification of the takeoff term of an indwelling treatment tool described later.

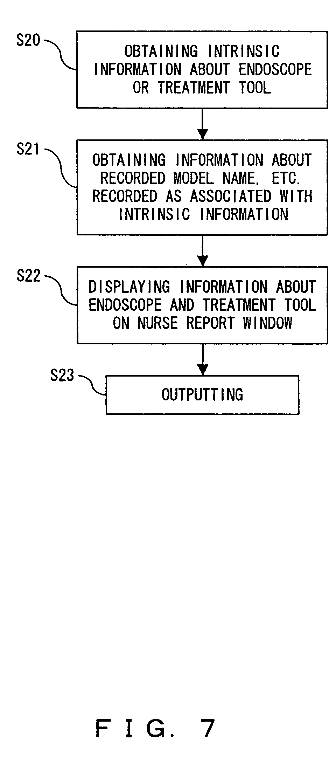

FIG. 7 shows a flow of the process of generating a nurse report. When the operator (nurse) opens the nurse report generation window 38 by selecting predetermined patient information using the image filing device 1, a part of the information (for example, patient ID, examination date, examination starting time, etc.) of the examination information about the patient information is transmitted to the server 4.

In the server 4, the received information (for example, patient ID, examination date, examination starting time, etc.) is stored in the storage device. The identification information about the scope or the treatment tool associated with the information is obtained by the CPU of the server (S20). Furthermore, the CPU of the server 4 obtains the information about the model name of the scope or the treatment tool stored as associated with the intrinsic information from the storage device (S21). Thus, the intrinsic information about the scope or the treatment tool and the information about the model name, etc. obtained by the server 4 are transmitted to the image filing device 1.

Upon receipt of the intrinsic information about the scope or the treatment tool, and the information about the model name, etc., the image filing device 1 automatically inputs the information in the input column on the nurse report generation window 38, and display it (S22). Then, the nurse input the necessary information in generating a report on the nurse report generation window 38, and enters the input information in the server 4.

The information input in S22 is output with the intrinsic information about the scope or the treatment tool entered as shown in FIG. 4 corresponding to the information (intrinsic information about the scope or the treatment tool in the information), and is effectively used. For example, it is transmitted to another system used in the hospital, used by the system, printed on the printer and used as a report, or used for a notification, etc. of takeoff term of the indwelling treatment tool described later (S23).

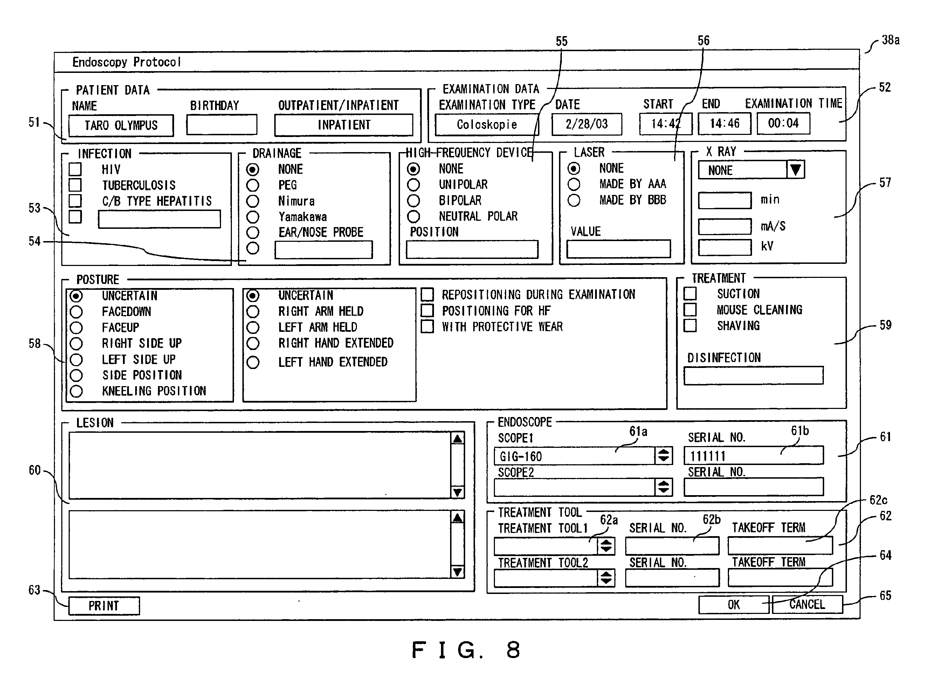

FIG. 8 shows an example of the nurse report generation window 38, and the window (endoscopy protocol window, and hereinafter referred to as a nurse report (during examination/treatment) generation window 38a) for generation of a nurse report during examination and treatment. The nurse report (during examination/treatment) generation window 38a includes a "patient data" column 51, an "examination data" column 52, an "infection" column 53, a "drainage" column 54, a "high frequency device" column 55, a "laser" column 56, an "X ray" column 57, a "posture" column 58, a "treatment" column 59, a "lesion" column 60, an "endoscope" column 61, a "treatment tool" column 62, a "printing" button 63, an "OK" button 64, and a "cancel" button 65.

In the "patient data" column 51 displays the name, the birthday, and outpatient/inpatient of a patient are displayed. In the "examination data" column 52, the examination type, the date of the day on which the examination was made (examination date), the examination starting time, the examination stop time, and the examination time are displayed. The diseases suffered by the patient can be input in the "infection" column 53. In the "drainage" column 54, the treatment applied to blocked bile duct, etc. can be selected. In the " high frequency device" column 55, the type of high frequency device can be selected.

In the "laser" column 56, the type of laser can be selected. In the "X ray" column 57, the information about the x ray to be used can be input. In the "posture" column 58, the posture of the patient during examination or treatment can be input. In the "treatment" column 59, the treatment (suction, mouse cleaning, shaving, disinfection, etc.) performed on the patient can be input. In the "lesion" column 60, the lesion information about the patient can be input.

The "endoscope" column 61 comprises a "scope 1 (or scope 2)" column 61a in which the scope name used in the examination or the treatment is input, and a "serial number" column 61b. The "treatment tool" column 62 comprises a "treatment tool 1 (or treatment tool 2)" column 62a for input of a treatment tool name used in the examination or the treatment, a "serial number" column 62b for input of the serial number, and a "takeoff term" column 62c for input of the takeoff term of the treatment tool.

In the "scope 1 (or scope 2)" column 61a and the "serial number" column 61b, the name of the scope and the serial number read by the ID detector 3 are automatically input as initial values on the examination execution window 33. The user can appropriately change the information about these items.

In the "treatment tool 1 (or treatment tool 2)" column 62a, the "serial number" column 62b, and the "takeoff term" column 62c, the scope name, the serial number, and the takeoff term read by the ID detector 3 on the examination execution window 33 are automatically input as initial values. The user can appropriately change the information about these items. The takeoff term is described later.

When the "printing" button 63 is pressed, the contents displayed and input on the nurse report (during examination/treatment) generation window 38a can be printed to reporting paper. When the "OK" button 64 is pressed, the contents input on the nurse report (during examination/treatment) generation window 38a can be entered in the server 4. At this time, the information input in the "scope 1 (or scope 2)" column 61a and the "serial number" column 61b is entered in the server 4 with at least the patient ID, the examination date, and the examination starting time associated with the information. The information input in the "treatment tool 1 (or treatment tool 2)" column 62a, the "serial number" column 62b, and the "takeoff term" column 62c is entered in the server 4 with at least the patient ID, the examination date, and the examination starting time associated with the information. When the "cancel" button 65 is pressed, the window terminates.

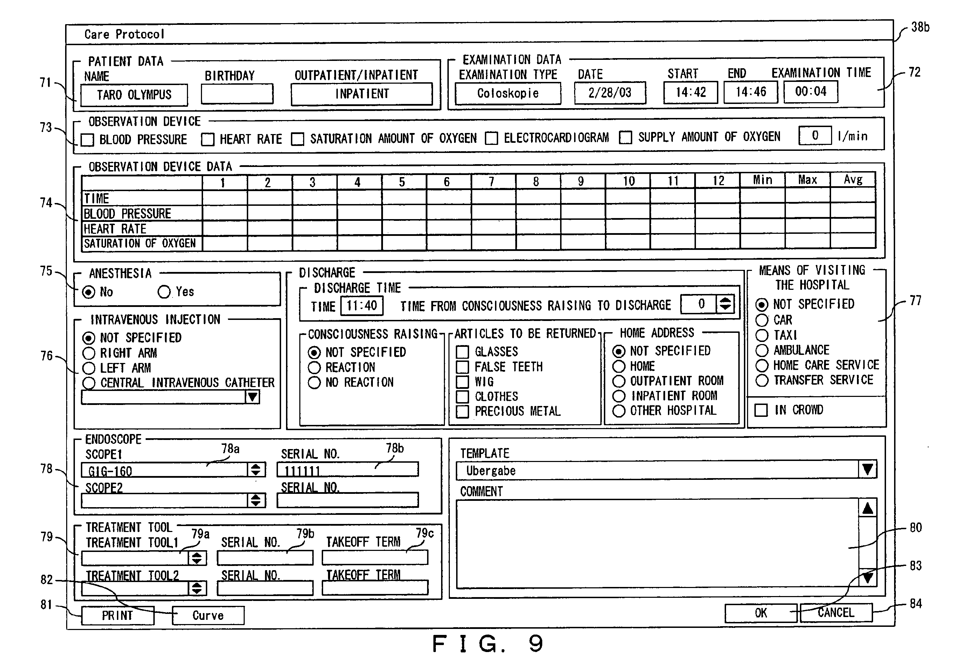

FIG. 9 shows an example of the nurse report generation window 38 used in generating a nurse report after examination or after treatment (hereinafter referred to as a nurse report (after examination/treatment) generation window 38b). In FIG. 9, the nurse report (after examination/treatment) generation window 38b comprises a "patient data" column 71, an "examination data" column 72, an "observation device" column 73, an "observation device data" list 74, an "anesthesia" column 75, an "intravenous injection" column 76, a "discharge" column 77, an "endoscope" column 78, a "treatment tool" column 79, a "comment" column 80, a "print" button 81, a "curve" button 82, an "OK" button 83, and a "cancel" button 84.

In the "patient data" column 71, the name, the birthday, and outpatient/inpatient of a patient are displayed. In the "examination data" column 72, the examination type, the date of the day on which the examination was made (examination date), the examination starting time, the examination stop time, and the examination time are displayed. In the "observation device" column 73, the type of the observation device used in observing the physiological condition of the patient such as the blood pressure, the heart rate, the saturation amount of oxygen, the electrocardiogram, the supply amount of oxygen, etc. can be input. The "observation device data" list 74 displays observation data obtained by the observation device.

In the "anesthesia" column 75, it is input whether or not anesthesia has been performed. In the "intravenous injection" column 76, it can be input to which region of the body of the patient the intravenous injection has been applied, or the central intravenous catheter has been applied. The "discharge" column 77 comprises a "discharge time" column to which the discharge time and the time from consciousness raising to discharge are input, a "consciousness raising" column in which the reaction of the patient at consciousness raising is input, an "articles to be returned" column for check of whether or not the articles received from the patient have been returned is input, a "home address" column to which the home address after examination is input, and "means of visiting the hospital" column to which the means of the patient visiting the hospital is input.

The "endoscope" column 78 comprises a "scope 1 (or scope 2)" column 78a for input of the scope name used in the examination or the treatment, and a "serial No." column 78b for input of the serial number. The "treatment tool" column 79 comprises a "treatment tool 1 (or treatment tool 2)" column 79a for input of the treatment tool name used in the examination or the treatment, a "serial No." column 79b for input of the serial number, and a "takeoff term" column 79c for input of the takeoff term of the treatment tool.

In the "scope 1 (or scope 2)" column 78a and the "serial No." column 78b, the name and the serial number of the scope read by the ID detector on the examination execution window 33 are automatically input as initial values. The user can appropriately change the information about these items.

The name, the serial number, and the takeoff term of the scope read by the ID detector on the examination execution window 33 are automatically input as the initial values to the "treatment tool 1 (or treatment tool 2)" column 79a, the "serial No." column 79b, and the "takeoff term" column 79c. The user can appropriately change the information about these items. The takeoff term is described later.

In the "comment" column 80, the format for output of a nurse report is selected from the "template" column to input a comment. When the "print" button 81 is pressed, the contents displayed and input on the nurse report (after examination/treatment) generation window 38b can be printed to the reporting paper.

When the "OK" button 83 is pressed, the contents input on the nurse report (after examination/treatment) generation window 38b can be entered in the server 4. At this time, the information input in the "scope 1 (or scope 2)" column 78a and the "serial No." column 78b is entered with at least the patient ID, the examination date, the examination starting time associated with the information.

Furthermore, the information input to the "treatment tool 1 (or treatment tool 2)" column 79a, the "serial No." column 79b, and the "takeoff term" column 79c is entered with at least the patient ID, the examination date, and the examination starting time associated with the information. When the "curve" button 82 is pressed, the number information displayed in the "observation device data" list 74 can be displayed as a graph.

When the "cancel" button 84 is pressed, the present window terminates.

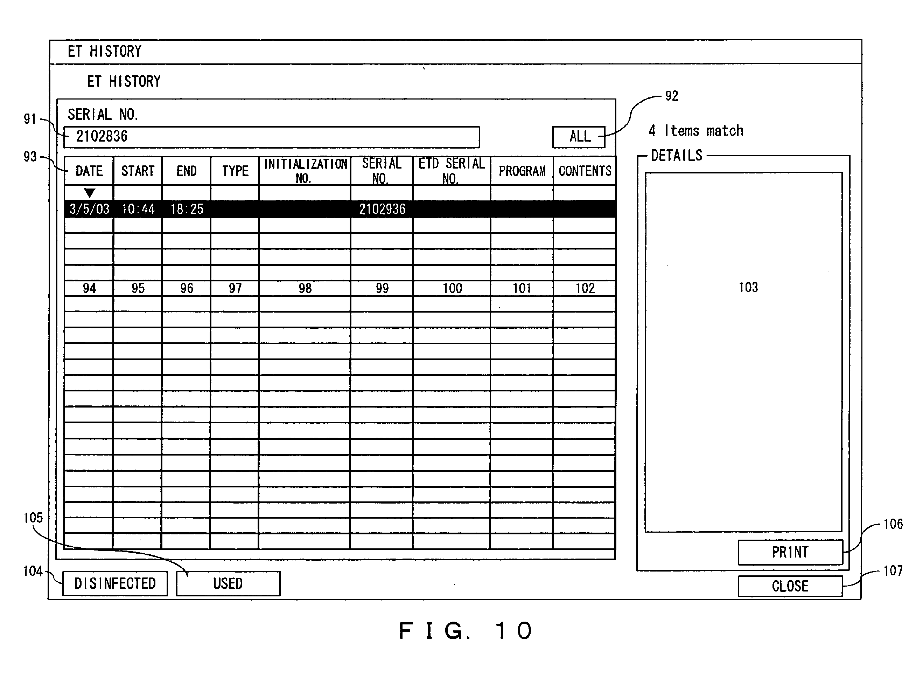

FIG. 10 shows a ET (endoscopy and therapy) history window, and this window is corresponding to the scope log window 39 or the treatment tool log window 40. When display of a scope log or display of treatment tool log is selected on the data management window 36, a transition is made to the ET history window. When a scope log is displayed and a treatment tool log is displayed, the same ET history window is used, but the displayed log information is different.

When a transition is made to the ET history window, the image filing device 1 transmits request information about transmitting a scope log or a treatment tool log to the server 4. The server 4 receives the request information, and the CPU of the server transmits the information about the scope log or the treatment tool log stored in the storage device of the server to the image filing device 1 according to the request information. After receiving the log information, the image filing device 1 displays the log information corresponding to the item in a predetermined item on the ET history window.

The ET history window comprises a "serial No." column 91, an "all" button 92, an "ET history list" 93, a "detail" column 103, a "disinfected" button 104, a "used" button 105, and a "close" button 107.

On the "ET history list" 93, the examination date on which the scope or the treatment tool is used is displayed in a "date" 94, the starting time of the examination and the treatment is displayed in a "start" 95, the end time of the examination and the treatment is displayed in an "end" 96, the type of the used scope or treatment tool is displayed in a "type" 97, the number of the scope or treatment tool set in the hospital is displayed in an "initialization No." 98, the serial number of the scope or the treatment tool is displayed in a "serial No." 99, the number of the scope or the treatment tool set for cleaning is displayed on an "ETD serial No." 100, the program of cleaning is displayed in a "program" 101, and the contents are displayed in a "contents" column 102.

In the "detail" column 103, detailed information can be displayed. The "serial No." column 91 is a retrieval input textbox in which the serial number is input. Only the ET history of the scope or the treatment tool corresponding to the input serial number is displayed on the "ET history list" 93. When the "all" button 92 is pressed, the retrieval is null, and all scopes and treatment tools are displayed on the "ET history list" 93.

When the "disinfected" button 104 is pressed, only the disinfected ET history information is displayed on the "ET history list" 93. When the "used" button 105 is pressed, only the used ET history information is displayed on the "ET history list" 93. When the "printing" button 106 is pressed, the "detail" column 103 can be printed. When the "close" button 107 is pressed, the present window can be terminated.

According to the present embodiment, the identification information about medical tools and the information about the examination and treatment can be associated with each other as stored. Therefore, the history of using medical tools can be easily traced, and the information can be effectively used. Furthermore, the identification (intrinsic) information about the endoscope or the treatment tool, etc. can be managed.

Additionally, when a report is generated, the intrinsic information about an endoscope and a treatment tool and the related information are automatically input on the report generation window, thereby efficiently generating a report, and reducing the load of generating a report.

As described above, since the intrinsic information about a used endoscope and treatment tool can be easily captured when an examination and treatment are performed, a nurse can reduce the laborious operation required in manually recording the information about a treatment tool and an endoscope in a report.

### <Second Embodiment>

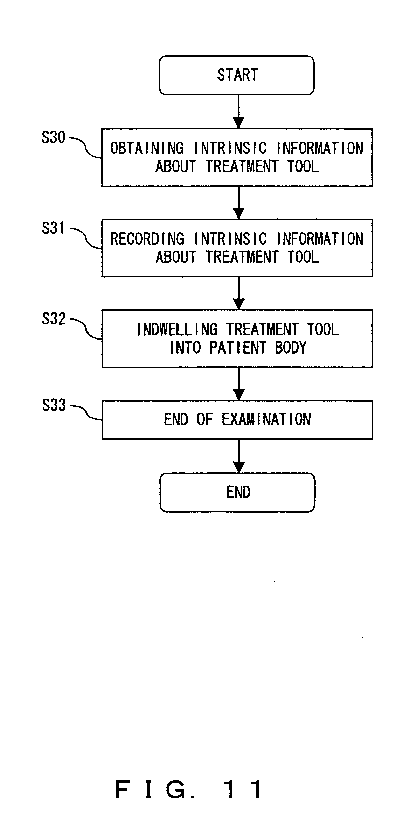

FIG. 11 shows a flow of the process of indwelling an indwelling treatment tool. The operation of the filing device 1 is basically similar to that shown in FIG. 6. First, the examination execution window 33 is opened, and the intrinsic information stored in the ID tag attached to the package of the indwelling treatment tool shown in FIG. 3C is read using the ID detector 3 (S30). The intrinsic information about the treatment tool read in S30 is stored in the data storage unit 10 of the image filing device 1 (S31). The indwelling treatment tool is indwelled in the body of a patient (S32). The information stored in the data storage unit 10 of the filing device 1 in S31 is entered in the server 4, thereby terminating the examination (S33).

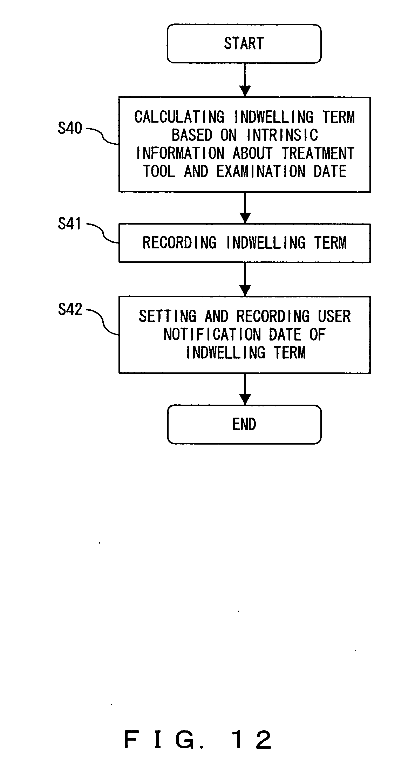

FIG. 12 shows the flow of setting the indwelling date performed by the server. First, the indwelling period is calculated based on the intrinsic information and the examination date about the indwelling treatment tool (S40). In this example, the indwelling period refers to a period set for each indwelling treatment tool, and a period in which the indwelling treatment tool can be dwelled in a human body. For example, 30 days are set for an indwelling treatment tool A, and 100 days are set for an indwelling treatment tool B. Since the storage device of the server 4 stores an indwelling period in advance (the initialization of the indwelling term is described later) for each indwelling treatment tool, the information about the indwelling period corresponding to the intrinsic information about the indwelling treatment tool entered by the CPU of the server as shown in FIG. 11 is obtained from the storage device of the server.

Furthermore, since the information entered as shown in FIG. 11 includes the examination date, the examination date is also obtained. The date obtained by adding the indwelling period to the obtained examination date is output as the indwelling term. Then, the indwelling term calculated in S40 is stored in the storage device of the server (S41).

Next, the user notification date of the indwelling term is set and stored (S42). The storage device of the server stores the relative number of days (or relative number of weeks) in advance (the initialization of the relative number of days (or relative number of weeks) is described later). The relative number of days refers to the day count information indicating the number of days before the indwelling term, for example, the values "-7", "-15", etc. If "relative number of days = -7", it indicates 7 days before the indwelling term. The relative number of days can be replaced with the relative number of weeks. In this case, "relative number of weeks = -4" indicates 4 weeks before the indwelling term.

The relative number of days is obtained from the storage device of the server, and the relative number of days is added to the indwelling term obtained above. The sum is stored in the storage device of the server as a user notification date for the indwelling term. That is, the user notification date is date information for notification of the indwelling term to a user (a doctor, a nurse, a patient, etc.) in advance.

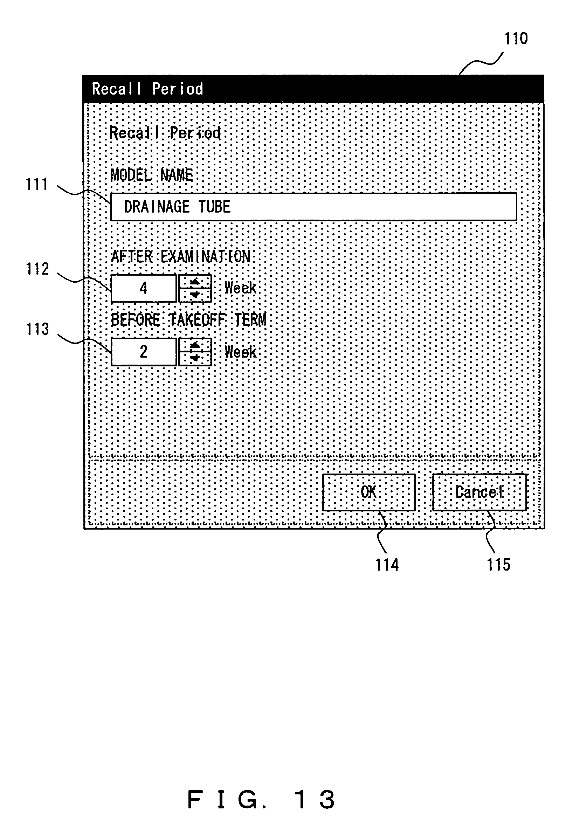

FIG. 13 shows the window for setting the takeoff term and the user notification date. A setting window (according to the present embodiment, it is a recall period window) 110 comprises a "model name" input column 111 for input of a model name of a treatment tool, an "after examination" input column 112 for input how many weeks after examination the treatment tool input to the "model name" input column 111 is to be taken off, a "before takeoff term" 113 for input how many weeks before takeoff term the user is to receive a notification, an "OK" button 114 for entry of the contents of the window in the server, and a "cancel" button 115 for termination of the current window.

First, the "model name" input column 111, the "after examination" input column 112, and the "before takeoff term" 113 of the indwelling treatment tool used in the examination and the treatment are input in advance on the window, and entered in the server. Then, in S40 shown in FIG. 12, the value input in the "after examination" input column 112 is used as the indwelling period for calculation of the indwelling term. On the setting window 110, "after examination = 4" is set, and the indwelling term is 4 weeks after the examination. As in S42 shown in FIG. 12, the "before takeoff term" 113 is used as the relative number of weeks. On the present setting window 110, "before takeoff term = 2", that is, two week before the takeoff term is the user notification date.

The indwelling term can be set for each indwelling treatment tool. When the user opens the nurse report (during examination/treatment) generation window 38a, the initial value of the takeoff term is entered in the server in the "takeoff term" column 62c. At this time, the "takeoff term" column 62c is a data item to which input can be made. Therefore, the user can arbitrarily enter a date in the server. The similar process can be performed in the "takeoff term" column 79c of the nurse report (after examination/treatment) generation window 38b.

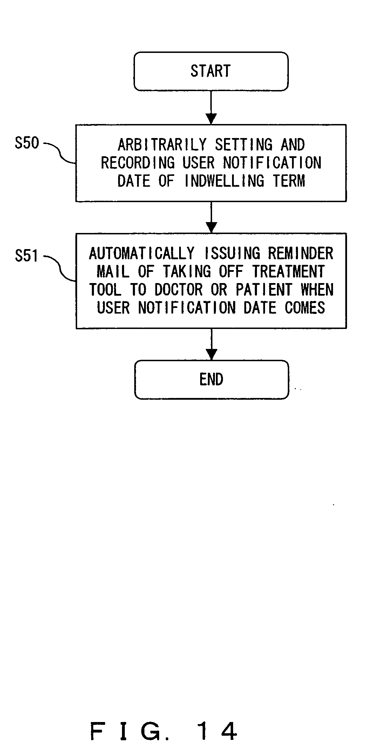

FIG. 14 shows the flow of the process until reminder mail is transmitted on the user notification date. The reminder mail is issued as a notification of an indwelling term, or a notification of the process of taking off an indwelling treatment tool because its indwelling term is coming up. The user notification date is set in the server in S42 shown in FIG. 12 (that is, set as the initial value), and the notification date can be arbitrarily changed (S50).

On the user notification date, the server 4 automatically issues reminder mail to the doctor, the nurse, or the patient (S51). The CPU of the server 4 performs the comparing process between the user notification date and the current date every day. If they matches, it determines that the user notification date has come, and issues the reminder mail.

Since the server stores the template information about the reminder mail depending on the destination, the name of the patient, the patient ID, the name of the hospital, the takeoff term, etc. can be assigned to the template. The contents of the reminder mail can be, for example, addressed to a patient as follows.
"Patient name: Taro Olympus
Patient ID: 11111111
Hospital name: XXXX Hospital
Notification: The takeoff date of the indwelling treatment tool is coming. Please visit the hospital by ....."

The reminder mail can be addressed in a listing format to a doctor as follows.

"Patient name: Taro Olympus, patient ID: 11111111, examination date:..., takeoff term: ...."

As the address of the reminder mail, the electronic mail addresses of the doctor in charge or a patient are stored in the storage unit of the server in advance. Therefore, based on the electronic mail addresses, reminder mail is transmitted to the doctor in charge or the patient. As a notification means, not only electronic mail, but also a post card, FAX, etc. can be used.

When a post card is used, the address of the destination of the doctor in charge or the patient, and the information about the coming takeoff date of the indwelling treatment tool are printed to the post card on a printer and mailed. When FAX is used, after once outputting the contents of the reminder mail on the printer, the mail can be transmitted to the doctor in charge or the patient by FAX, or can be transmitted directly from the server 4.

As described above, the indwelling term of the indwelling treatment tool can be calculated according to the intrinsic information about the used treatment tool, and automatically announced to the patient or the doctor, thereby preventing the indwelling treatment tool from being remained in the patient after the takeoff date, and reducing the trouble of notifying the patient of the takeoff date by manual labor.

Furthermore, when a nurse generates a report, the intrinsic information about a scope or a treatment tool and the information about the model name, etc. are automatically input to the corresponding position on the report generation window. Therefore, the report can be efficiently generated, and the load of generating the report can be reduced. Since the nurse report can be generated during examination or during treatment, it is especially effective.

As mentioned above, according to the present invention, the intrinsic information about an endoscope or a treatment tool can be easily stored with the examination information. As a result, the operation performed when the intrinsic information is input can be reduced, and the information about the treatment tool or the scope used during examination can be easily traced.

Furthermore, the stored intrinsic information about the endoscope or the treatment tool, and the examination information can be effectively used. Therefore, for example, the takeoff term of the indwelling treatment tool can be announced to the user without fail in advance.

Additionally, when a report is generated, the intrinsic information about an endoscope or a treatment tool, and the related information can be automatically input on the report generation window, and the report can be efficiently generated, thereby successfully reducing the load of generating a report.

## Claims

1. A medical system for managing a medical tool, comprising:
an identification information obtaining unit (3) obtaining identification information about a medical tool stored in information resources provided for the medical tool;
a medical practice information obtaining unit (1) obtaining medical practice information about the medical practice performed using the medical tool; and
a storage control unit (4) storing medical tool and practice related information which refers to association between the identification information obtained from the identification information obtaining unit and the medical practice information obtained from the medical practice information obtaining unit.

2. The system according to claim 1, further comprising
a notification unit (4) notifying of an expiration date of the medical tool in advance according to the medical tool and practice related information stored in the storage control unit (4).

3. The system according to claim 1, wherein:
the information resources are transponder; and
the identification information obtaining unit (3) is a reader for reading information stored in a storage unit in the transponder.

4. The system according to claim 2, wherein
the notification unit (4) comprises:
a service period related information obtaining unit (S40,S41) obtaining, in the medical tool and practice related information stored by the storage control unit, medical tool service period information which is information about a period in which the medical tool can be used, and medical practice date information which is information about a date on which the medical practice is performed; and
a calculation unit (S42) calculating notification date information which is information about a date on which an expiration date of the medical tool is announced in advance according to the medical tool service period information and the medical practice date information obtained by the service period related information obtaining unit, and information about a predetermined period;
a comparison unit (S51) comparing the notification date information with information about a current date; and
a transmission unit (S51) transmitting information about the medical tool expiration date based on a comparison result by the comparison unit.

5. The system according to claim 1, wherein
the medical tool is at least one of an endoscope and a treatment tool for examination or treatment of an affected part of a patient.

6. The system according to claim 1, wherein
the medical tool is a treatment tool indwelled in a body of a patient.

7. The system according to claim 1, further comprising
output unit (35) outputting the identification information and the medical practice information stored by being associated by the storage control unit, and information about the medical tool corresponding to the identification information.

8. The system according to claim 7, wherein
the output unit (35) outputs the identification information, the medical practice information, and the information about the medical tool as a report for a doctor or a nurse.

9. The system according to claim 1, further comprising:
a report generation unit (35) generating a report for the medical practice; and
an automatic input unit automatically inputting in a corresponding position on the report generation window the identification information or information about a medical tool obtained according to the identification information when a report of the medical practice is generated by the report generation unit.

10. The system according to claim 7, wherein
the information about the medical tool comprises at least one of a production number, a model name, and a manufacturer name of the medical tool.

11. The system according to claim 9, wherein
the information about the medical tool comprises at least one of a production number, a model name, and a manufacturer name of the medical tool.

12. A medical server system (4) for managing a medical tool, comprising:
a storage unit (S12) storing medical tool and practice related information about the association between the information about the medical tool and the information about the medical practice performed using the medical tool; and
a notification unit (S51) notifying of an expiration date of the medical tool in advance according to the medical tool and practice related information stored in the storage control unit.

13. The system according to claim 12, wherein
the notification unit comprises:
a service period related information obtaining unit (S40,S41) obtaining, in the medical tool and practice related information, medical tool service period information which is information about a period in which the medical tool can be used, and medical practice date information which is information about a date on which the medical practice is performed; and
a calculation unit (S42) calculating notification date information which is information about a date on which an expiration date of the medical tool is announced in advance according to the medical tool service period information and the medical practice date information obtained by the service period related information obtaining unit, and information about a predetermined period;
a comparison unit (S51) comparing the notification date information with information about a current date; and
a transmission unit (S51) transmitting information about the medical tool expiration date based on a comparison result by the comparison unit.

14. A medical method for managing a medical tool, comprising:
obtaining identification information about a medical tool stored in information resources provided for the medical tool (S11);
obtaining medical practice information about the medical practice performed using the medical tool (S12,S13,S14); and
storing medical tool and practice related information which refers to association between the identification information and the medical practice information (S12,S13,S14).

15. The method according to claim 14, further comprising
notifying of an expiration date of the medical tool in advance according to the medical tool and practice related information (S17,S51).

16. The method according to claim 15, wherein
when the notification is issued,
obtaining, in the medical tool and practice related information, medical tool service period information which is information about a period in which the medical tool can be used, and medical practice date information which is information about a date on which the medical practice is performed (S40,S41); and
calculating notification date information which is information about a date on which an expiration date of the medical tool is announced in advance according to the medical tool service period information, the medical practice date information, and information about a predetermined period (S42);
comparing the notification date information with information about a current date (551); and
transmitting information about the medical tool expiration date (S51).

17. The method according to claim 14, wherein
the medical tool is at least one of an endoscope and a treatment tool for examination or treatment of an affected part of a patient.

18. The method according to claim 14, wherein
the medical tool is a treatment tool indwelled in a body of a patient.

19. The method according to claim 14, further comprising
outputting the medical tool and practice related information in which the identification information is associated with the medical practice information, and the medical tool information about the medical tool corresponding to the identification information (S15,S16,S17).

20. A medical system for managing a medical tool, comprising:
identification information obtaining means (3) for obtaining identification information about a medical tool stored in information resources provided for the medical tool;
medical practice information obtaining means (1) for obtaining medical practice information about the medical practice performed using the medical tool; and
storage control means (4) for storing medical tool and practice related information which refers to association between the identification information obtained from the identification information obtaining means and the medical practice information obtained from the medical practice information obtaining means.

21. A medical server system (4) for managing a medical tool, comprising:
storage means (S12) for storing medical tool and practice related information about the association between the information about the medical tool and the information about the medical practice performed using the medical tool; and
notification means (S51) for notifying of an expiration date of the medical tool in advance according to the medical tool and practice related information stored in the storage control means.

22. A computer-readable storage medium storing a medical program used to direct a computer to perform the process for managing a medical tool, comprising:
an identification information obtaining process (S11) of obtaining identification information about a medical tool stored in information resources provided for the medical tool;
a medical practice information obtaining process (S12,S13,S14) of obtaining medical practice information about the medical practice performed using the medical tool; and
a storage control process (S12,S13,S14) of storing medical tool and practice related information which refers to association between the identification information obtained in the identification information obtaining process and the medical practice information obtained in the medical practice information obtaining process.

23. The storage medium according to claim 22, further comprising
notifying process (S51) of notifying of an expiration date of the medical tool in advance according to the medical tool and practice related information stored in the storage control process.

24. The storage medium according to claim 22, wherein
the medical tool is at least one of an endoscope and a treatment tool for examination or treatment of an affected part of a patient.

25. The storage medium according to claim 22, wherein
the medical tool is a treatment tool indwelled in a body of a patient.

26. The storage medium according to claim 22, further comprising:
a report generation process (S15,S16) of generating a report for the medical practice; and
an automatic input process of automatically inputting in a corresponding position on the report generation window the identification information or information about a medical tool obtained according to the identification information when a report of the medical practice is generated in the report generation process.

27. A computer data signal embodied in a carrier wave for management of a medical tool, used to direct a computer to perform:
an identification information obtaining process (S11) of obtaining identification information about a medical tool stored in information resources provided for the medical tool;
a medical practice information obtaining process (S12,S13,S14) of obtaining medical practice information about the medical practice performed using the medical tool; and
a storage control process (S12,S13,S14) of storing medical tool and practice related information which refers to association between the identification information obtained in the identification information obtaining process and the medical practice information obtained in the medical practice information obtaining process.

28. A medical program product used to direct a computer to perform a process for managing a medical tool, comprising:
an identification information obtaining process (S11) of obtaining identification information about a medical tool stored in information resources provided for the medical tool;
a medical practice information obtaining process (S12,S13,S14) of obtaining medical practice information about the medical practice performed using the medical tool; and
a storage control process (S12,S13,S14) of storing medical tool and practice related information which refers to association between the identification information obtained in the identification information obtaining process and the medical practice information obtained in the medical practice information obtaining process.
